Europäisches Patentamt

European Patent Office

Office européen des brevets

(19)

(11) Publication number: **0 441 071 B1**

# EUROPEAN PATENT SPECIFICATION

(12)

(45) Date of publication of patent specification: **21.09.94**  (51) Int. Cl.5: **C12N 15/00**, //A61K39/112

(21) Application number: **90400326.6**

(22) Date of filing: **06.02.90**

(54) **Transformed shigella.**

(43) Date of publication of application:
**14.08.91 Bulletin 91/33**

(45) Publication of the grant of the patent:
**21.09.94 Bulletin 94/38**

(84) Designated Contracting States:
**AT BE CH DE DK ES FR GB GR IT LI LU NL SE**

(56) References cited:
**EP-A- 0 351 322**

**INFECTION AND IMMUNITY, vol. 57, no. 7, July 1989, pages 2136-2140, American Society for Microbiology; C.J. DORMAN et al.: "Characterization of porin and ompR of a virulent strain of Salmonella typhimurium: ompR mutants are attenuated in vivo"**

**J. BIOCHEM., vol. 101, 1987, pages 387-396; T. MIZUNO et al.: "Isolation and characterization of deletion mutants of ompR and envZ, regulatory genes for expression of the outer membrane proteins OmpC and OmpF in Escherichia coli"**

(73) Proprietor: **INSTITUT PASTEUR**
**25-28, rue du Docteur Roux**
**F-75724 Paris Cédex 15 (FR)**

Proprietor: **INSTITUT NATIONAL DE LA SANTE ET DE LA RECHERCHE MEDICALE (INSERM)**
**101, rue de Tolbiac**
**F-75654 Paris Cédex 13 (FR)**

(72) Inventor: **Sansonetti, Philippe**
**131 Bld. Brune**
**F-75014 Paris (FR)**
Inventor: **Bernardini Maria Lina**
**247 rue de Vaugirard**
**F-75015 Paris (FR)**
Inventor: **Fontaine, Annick**
**18 rue Liancourt**
**F-75014 Paris (FR)**

(74) Representative: **Gutmann, Ernest et al**
**Ernest Gutmann - Yves Plasseraud S.A.**
**3, rue Chauveau-Lagarde**
**F-75008 Paris (FR)**

CHEMICAL ABSTRACTS, vol. 110, no. 5, 30th
January 1989, page 227, abstract no. 35607v,
Columbus, Ohio, US; S.A. BENSON et al.:
"Mutations that alter the pore function of the
OmpF porin of Escherichia coli K12", & J.
MOL. BIOL. 1988, 203(4), 961-70

INFECTION AND IMMUNITY, vol. 55, no. 3,
March 1987, pages 594-599, American Soci-
ety for Microbiology; K.M. LAWLOR et al.:
"Virulence of iron transport mutants of
Shigella flexneri and utilization of host iron
compounds"

VACCINE, vol. 7, no. 5, October 1989, pages
443-450, Butterworth & Co. (Publishers) Ltd,
Guildford, Surrey, GB; P.J. SANSONETTI et
al.: "Construction and evaluation of a double
mutant of Shigella flexneri as a candidate
for oral vaccination against shigellosis"

**Description**

This invention relates to a method of modifying the genome of an entero-invasive wild strain of Shigella, such as S. flexneri, so that the ability of the strain to invade epithelial cells, and thereby infect a host, is subtantially reduced. This invention particularly relates to such a modified strain of Shigella which can be used to immunize a host against the wild strain of Shigella.

Shigellosis or bacillary dysentery is a disease that is endemic throughout the world. The disease presents a particularly serious public health problem in tropical regions and developing countries where Shigella dysenteriae 1 and S. flexneri predominate. In industrialized countries, the principal etiologic agent is S. sonnei although sporadic cases of shigellosis are encountered due to S. flexneri, S. boydii and certain entero-invasive Escherichia coli.

Shigella are pathogens which cause bacillary dysentery by penetrating and replicating within human colonic epithelial cells (2). This invasive process causes ulcerative lesions which result in a bloody and purulent diarrhea characteristic of bacillary dysentery. The invasive phenotype of Shigella can be studied by laboratory methods using cell culture models. Virulent Shigellae are able to penetrate HeLa cells and cause a cytopathic effect on a confluent monolayer (plaque assay)(3). Animal models include the Sereny test which assesses the virulence of Shigella by measuring the ability of invasive bacteria to cause a keratoconjunctivitis in guinea pigs (4). Although expression of genes located on the chromosome are required for full virulence (5), genetic loci encoding entry into epithelial cells (6), as well as inter- and intra-cellular spread (7,8), are located on a 220 kb virulence plasmid (9).

Shigellae are bacteria that utilize the fecal-oral route for transmission. In order to cause the disease, infecting organisms have to adapt to different conditions while moving from a free-living state to a host-associated state. In this process, the bacteria must respond to a wide variety of external stimuli including changes in temperature, pH, osmolarity, nutrient deprivation and competitive growth. It has been recently demonstrated that many bacterial virulence factors are coordinately regulated by two-component transcriptional regulators which respond to environmental stimuli (10). The deduced amino acid sequences of such proteins are highly homologous to the ompR-envZ genes that are required in E. coli for the osmo-dependent transcriptional regulation of expression of the ompF and ompC genes (11,12). Shigellae seem to have evolved at least two specialized systems which regulate the expression of virulence genes in response to environmental stimuli. The invasive phenotype is expressed at 37° but not at 30°C. virR, a chromosomal transcriptional regulator gene, represses the expression of virulence genes (at 30°C) (13).

Dorman et al (1989) Infection and Immunity 57(7), 2136-2140 disclose ompR mutants of Salmonella thyphimurium and that such mutants protected orally immunized mice against the wild-type Salmonella strain.

Summary of the Invention

In accordance with this invention, a method is provided for making a vaccine against a wild strain of an entero-invasive Shigella, such as S. flexneri, by providing a mutant of the Shigella strain having a modified genome, the method being characterized by the step of:

mutagenizing the ompB locus of the strain so that at least a portion of its ompR gene and at least a portion of its envZ gene are removed or permanently inactivated.

Also in accordance with this invention, the genome of the wild strain of Shigella is further modified by mutagenizing at least one additional gene encoding a protein necessary for the strain to: a) invade and then multiply within a cell of an infected host; b) spread substantially within infected cells and then from infected to uninfected cells of the host; and/or c) produce toxins which will kill subtantial numbers of the host's infected, as well as uninfected, cells.

Further in accordance with this invention are provided vaccines containing such Shigella mutants which are useful against the wild strain of Shigella.

Brief Description of the Drawings

Fig. 1A    shows schematically the in vitro deletion of the entire envZ gene and most of the ompR gene from the ompB locus from E. coli and the subcloning of the remainder of the ompB locus into the suicide vector PJM703-1 (18) in Example 1. Only relevant restriction sites are shown.

Fig. 1B    shows a restriction map of the BamHI 7.8 kb fragment from E. coli, containing the ompR-envZ operon (21) and flanking sequences before and after in vitro deletion of the envZ gene and most of the ompR gene in Example 1.

Fig. 2    shows an immunoblot analysis of virulence-associated peptides found in whole cell S. flexneri lysates separated by polyacrylamide gel electrophoresis and probed with serum obtained from a monkey convalescing from shigellosis; lane 1: M90T; lane 2: M90TX; lane 3: M90TX carrying pAT003; and lane 4: SC437. M90TX is a spontaneously occurring envZ mutant which is not part of the invention but is used as a control.

Detailed Description of the Invention

A method is provided for modifying a wild strain of an entero-invasive Shigella, such as S. dysenteriae 1 or S. flexneri, so that the modified strain can be used for making a vaccine against the wild strain of Shigella. The resulting modified strain is sufficiently active to protect mammals, such as humans, against the wild strain of Shigella but is not too invasive in mammals, so that the strain can safely be used in a vaccine.

The wild strain of Shigella is modified in accordance with this invention so that its ability to invade epithelial cells and thereby infect cells of a host, particularly a human host, is significantly reduced. This is accomplished by transforming the genome of the wild strain of Shigella, so that:

a) in the chromosomal ompB locus, both the ompR gene and the envZ gene are wholly or partly removed from the genome of the wild strain or are permanently inactivated, and preferably at least significant portions of the ompR and envZ genes are removed from the genome of the wild strain; or

b) the chromosomal ompF gene is wholly or partly removed from the genome of the wild strain or permanently inactivated, and preferably at least a significant portion of the ompF gene is removed from the genome of the wild strain.

It has now been discovered that in Shigella, particularly S. flexneri, either both the ompR and envZ genes or the ompF gene can be mutagenized in order to modulate the expression of the vir genes responsible for invasion of epithelial cells. Without such mutagenized ompR and envZ genes or mutagenized ompF gene, vir-gene expression by the Shigella would be markedly enhanced under conditions of high osmolarity (300 mOsm) such as occurs both extra intra- and intra-cellularly in tissue.

As described below, ompB mutants and ompF mutants of this invention have significantly decreased virulence in low and high osmolarity conditions. An envZ::Tn10 mutant remained invasive, but its virulence was significantly decreased due to its inability to survive intra-cellularly. Using a vir::lac operon fusion, this mutation was shown to decrease $\beta$-galactosidase expression in low, as well as in high, osmolarity conditions but did not affect vir expression in response to changes in osmolarity. A ompB deletion mutant of this invention, constructed via allelic exchange with an in vitro mutagenized ompB locus of E. coli. This mutation severely impaired virulence and rendered a vir::lac fusion mutant unable to express $\beta$-galactosidase both in low and high osmolarity conditions. Therefore, as already observed in other bacterial pathogens, a two-component regulatory system modulates virulence according to environmental conditions. In addition, a spontaneous avirulent variant of S. flexneri serotype 5, M90T, isolated in 1965 by Formal et al (1), has been mapped at the ompB locus since transcomplementation with the E. coli ompB locus restored complete virulence. Introduction of the vir::lac fusion into this mutant did not result in the expression of $\beta$-galactosidase (Lac⁻). Osmoregulated Lac⁺ revertants were isolated at a frequency of 5 x $10^{-7}$, thus suggesting that a phase variation phenomenon may exist in S. flexneri.

The wild strain of entero-invasive Shigella preferably is also genetically transformed in accordance with this invention so that the resulting mutant strain is substantially incapable of: a) invading and then multiplying substantially within cells of an infected host; b) spreading substantially within infected cells and from infected to uninfected cells of the host; and/or; c) producing toxins which will kill substantial numbers of the host's infected, as well as uninfected, cells. It is preferred that this be accomplished by transforming one or more, additional genes in the genome of the wild strain in accordance with the disclosure in European patent application 89402024.7, filed July 13, 1989 (published as European patent publication 0,351,322), which is incorporated herein by reference.

Preferably, the additional gene, to be transformed in the genome of the wild strain of Shigella, is a gene coding for one or more proteins, necessary for the strain to spread within and between the infected host's cells, i.e., the icsA gene. This additional gene is also mutagenized by wholly or partly removing the gene from the genome of the wild strain or permanently inactivating the gene, preferably by removing at least significant portions of the gene from the genome of the wild strain. In this regard, one or more further genes of the wild strain (i.e., in addition to the icsA gene) can also be mutagenized if necessary, such as a gene encoding a protein necessary for the chelation and/or transport of iron in the strain (e.g., an enterobactin or enterochelin gene of S. dysenteriae 1 or an aerobactin gene of S. flexneri). This can be accomplished by wholly or partly removing the further gene(s) from the genome of the wild strain or permanently inactivating

4

the further genome. For a wild strain such as a S. dysenteriae 1, it may also be necessary that all or part of the gene(s), preferably just the A subunit gene, coding for Shiga-toxin, also be removed from the genome of the wild strain.

In carrying out this invention, genes of the wild strain of Shigella can be wholly or partly removed or permanently inactivated in a conventional manner. For example, the genes of the wild strain can be subjected to allelic exchange with in vitro mutagenized genes which come from the wild strain, itself, or from other entero-invasive Shigellae or E. coli (that are known to contain highly homologous ompR, envZ, ompF, and icsA genes (11, 12)). Preferably, such in vitro mutagenized genes have at least significant portions thereof removed or inactivated. In this regard, it is preferred that the mutagenized genes not be simply inactivated by means of transposons which are inserted into the genes and which can be lost by the genes when they are reproduced in vivo in subsequent Shigella generations when making vaccines of this invention. Rather, the mutagenized genes preferably have had at least significant portions thereof deleted, and suitable vaccine-compatible marker genes are preferably inserted within such deletions. Such marker genes permit so-transformed Shigella to be easily identified. The preferred marker genes are the heavy metal-resistance genes such as the mercury, arsenate, arsenite, antimony, cadmium, zinc and/or cobalt-resistance genes.

The cells of the modified Shigella strain of this invention can be cultured and then attenuated in a conventional manner. The cells can then be mixed with conventional pharmaceutically acceptable vehicles (e.g., an aqueous saline solution) and optionally with conventional excipients (e.g., a pharmaceutically acceptable detergent) to form a vaccine against the wild strain. The vaccine can be formulated to contain a final concentration of cell material in the range of 0.2 to 5 mg/ml, preferably 0.5 to 2 mg/ml. After formulation, the vaccine can be placed in a sterile container which is then sealed and stored at a low temperature (e.g., 4°C), or it can be freeze dried.

In order to induce immunity in a human host to a wild strain of Shigella, one or more doses of the vaccine, suitably formulated, can be administered in doses containing about $10^8$-$10^{10}$, preferably about $10^9$, lyophilized Shigella cells. The vaccine can be administered orally in a conventional manner. The treatment can consist of a single dose of vaccine or a plurality of doses over a period of time.

The Examples, which follow, illustrate this invention.

EXAMPLES

Unless otherwise indicated, the cloning and transformation procedures and techniques used in the Examples are the same as are generally described in Maniatis et al, "Molecular Cloning -- A Laboratory Manual", Cold Spring Harbor Laboratory (1982).

Bacterial Strains and Media

The bacteria and plasmids, used in the Examples, are set forth in Table I. Bacteria were routinely cultured in tryptic soy broth (Diagnostics Pasteur, Marnes la Coquette, France) or L broth (23). The ability to bind the pigment Congo red was assessed on tryptic soy broth plates containing 1.5% agar and 0.01% Congo red. M9 salts (23) were used for preparing minimal medium. Carbon sources were added to a final concentration of 0.2%, and the medium was supplemented with 10ug per ml of nicotinic acid to allow growth of Shigella. When necessary, casaminoacids were added at 100ug/ml. Ampicillin, kanamycin, spectinomycin and tetracycline were added at 100, 50, 100 and 10 ug per ml respectively.

Genetic Procedures

Transformations were performed as described by Dagert and Ehrlich (24). Generalized transduction was with bacteriophage P1 as described by Miller (23). All experiments studying expression of the vir::lac and ompF'$^-$lacZ$^+$ gene fusions were performed in medium A (25) supplemented with different concentrations of sucrose (5, 10, 15%) or in T. Y. G. medium (10 g tryptone, 5 g yeast extract and 0.4 g D-glucose per liter) with the addition of KC1 (0.3 and 0.6 M). The $\beta$-galactosidase assay was performed by the method of Miller (23).

Virulence Assays

The HeLa cell invasion assay was performed as described by Hale and Formal (26). The other assays used to measure virulence were the plaque assay described by Oaks et al (3) and the Sereny test (4).

### Western Blot Hybridization

Immunoblotting procedures were carried out as described by Burnette (27) using the serum of a monkey experimentally infected with the wild type S. flexneri isolate, M90T.

### DNA manipulations

Plasmid DNA was isolated using the alkaline lysis method (28). Total DNA extraction was performed as described by Silhavy et al (29). Restriction endonucleases and DNA T4 ligase were used according to the manufacturers' recommendations.

### Example 1: Construction of plasmids pHS6200, pHS6201 and pHS6202

### Construction of plasmid pHS6200

Plasmid pAT003 (21) contains the entire ompR-envZ operon of E. coli K-12 on a 7.8 kb BamHI fragment cloned into the BamHI site of pBR322. This fragment has a unique EcoRI site inside the envZ coding sequence. In order to take advantage of this unique EcoRI site on plasmid pAT003, the EcoRI site of pBR322 had to be removed. pAT003 was therefore partially digested with EcoRI to obtain a linear plasmid. These linear molecules were then treated with the Klenow enzyme, ligated and transformed into MC1061. Transformants were screened for loss of the pBR322 EcoRI site. Plasmid pHS6200 was selected for further experiments (Fig. IA).

### Construction of Plasmid pHS6201

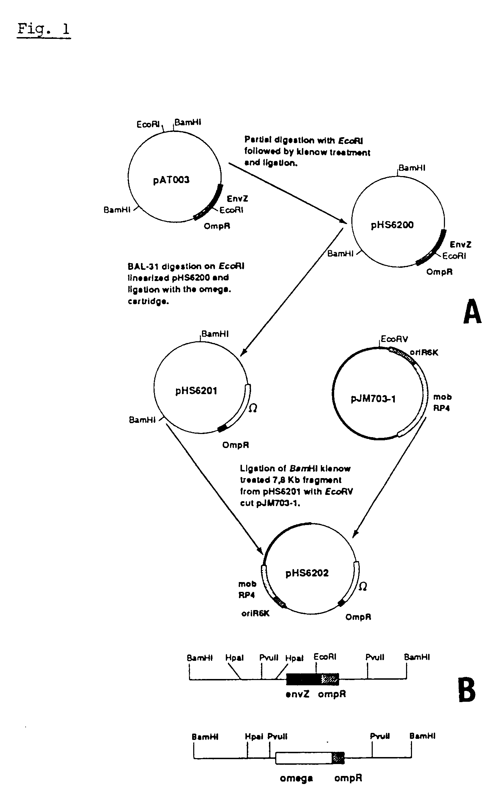

In vitro deletion of the ompR and envZ coding sequences was achieved by cleaving pHS6200 with EcoRI and treating the linear molecule with BAL-31 nuclease. Molecules which had lost about 2 kb were electro-eluted from an agarose gel and ligated on the interposon, omega (30), which had been purified as a 2kb SmaI fragment. Omega encodes spectinomycin resistance and is flanked by T4 translation transcription stop signals. DNA from 20 spectinomycin resistant transformants was cleaved with BamHI in order to estimate the size of the deletions made by BAL-31 in the ompR-envZ operon. Plasmid pHS6201, which contained a deletion of approximately 2 kb in the ompR-envZ operon, was kept for further experiments (Fig. 1A). From restriction mapping data, pHS6201 was found to have lost all of envZ coding sequence and most of ompR (Fig. 1B).

### Construction of pHS6202

The 7.8 kb BamHI fragment from pHS6201, containing omega and the flanking sequences of ompR and envZ, was isolated and ligated with EcoRV linearized pJM703.1 (18), a suicide vector which has previously been used for similar experiments (31). Strain SM10 pir (18) was transformed with this ligation and plated on medium containing spectinomycin. The resulting plasmid, pHS6202, was used for in vivo mutagenesis of M90T (20). See Fig. 1A and Example 4, below.

### Nick translation and hybridization

DNA fragments, to be used as probes in Example 4, below, were labelled by nick translation with [32]p-labelled 5'dCTP (Amersham). Three probes were labelled: a) a 7.8 kb BamHI fragment from pAT003 containing the entire ompR-envZ operon and flanking sequences; b) a 1.4 kb HpaI-EcoRI fragment containing internal coding sequence of envZ (Fig. 1B); and c) the interposon, omega, as a 2 kb EcoRI fragment. Total DNA from M90T and SC433 was digested with BamHI and loaded on a 0.7% agarose gel. DNA was transferred from the gel to nitrocellulose filter by the method of Southern (32). Hybridization was carried out at 65°C overnight, and washing was performed at 65°C in 6x SSC (1 x SSC: 0.15 M Nacl and 0.015 M sodium citrate).

### Example 2: Determining whether S. flexneri vir gene expression is modulated by changes in osmolarity

In order to assess whether expression of invasion genes was affected by changes in osmolarity, a P1 lysate of BS184 (vir-83::MudI1734) was used to transduce a vir::lac operon fusion to S. flexneri 5 wild type M90T, thus generating strain SC 430 (Lac+KmR). As expected, SC430 was unable to invade HeLa cells and was avirulent in the Sereny test. Like BS184, SC430 produced detectable levels of $\beta$-galactosidase. When SC430 was grown in high osmolarity medium (i.e., 15% sucrose), the $\beta$-galaotosidase activity was three to four fold higher than that observed when grown in a low osmolarity medium (Table 2). These data indicate that S. flexneri vir gene expression was regulated in response to osmolarity, with an increased response in high osmolarity conditions.

Example 3: Determining whether mutation in envZ reduces the virulence of S. flexneri and affects its capacity to respond to changes in osmolarity

Using P1 transduction, an envZ::Tn10 mutation was transferred from E. coli JM60 (15) to M90T (20) and SC430, thus generating strains SC432 and SC431 respectively (Table 1).

The ompB locus is located at 75 min on the chromosome of E. coli, very close to the malA locus (75 min). Wild type isolates of S. flexneri do not ferment maltose. Both transductants that had received the ompB region of E. coli also expressed a Mal+ phenotype on MacConkey Maltose plates.

SC432 was tested for expression of the various virulence phenotypes, including the ability to bind the dye Congo red (Pcr phenotype). The loss of Congo red binding (Pcr⁻) is correlated with the loss of invasive ability (33). SC432 showed a Pcr+ phenotype, it was able to penetrate into HeLa cells (Table 4), but its rate of invasion of HeLa cells, as well as of intra-cellular multiplication, was severely reduced by the envZ mutation. This mutant did not form plaques on a confluent monolayer of HeLa cells and provoked a mild and delayed response in the Sereny test (Table 4). A slight and transient conjunctivitis without purulent exudate was observed after 72 hours instead of the severe keratoconjunctivitis which was observed with wild type M90T after 24 hours. Immunoblotting, used to test the mutant strain for expression of the four virulence-associated immunogenic peptides (Ipa proteins) encoded by the virulence plasmid, showed that it produced wild type levels of these proteins (data not shown) (Table 4). Complementation tests performed with plasmid pAT003, carrying the E. coli ompR-envZ cloned genes, restored a fully virulent phenotype on SC432.

SC431 (KmR, TcR) produced low levels of $\beta$-galactosidase (Table 2), but the $\beta$-galactosidase activity increased approximately by four fold in high osmolarity medium. From this, it can be concluded that, although inactivation of envZ affects the virulence of Shigella, the envZ product is only required for basic expression of vir genes. Indeed, the induction of vir under high osmolarity conditions does not require envZ.

The envZ::Tn10 mutation caused a marked reduction in Shigella virulence as measured by virulence tests. Nevertheless, the bacteria were still able to invade HeLa cells and elicit a weak positive Sereny test. In the E. coli ompR-envZ operon, transcription goes from the ompR gene to the envZ gene. So, it can be assumed that the ompR gene was still expressed in SC432.

Example 4: Construction and characterization of a ΔompB mutant of M90T

In order to test the role of both ompR and envZ genes in Shigella virulence, a ΔompB mutant of S. flexneri was constructed. The plasmid pHS6202 was transferred into M90T by conjugation. After conjugation, bacteria were plated on minimal medium containing 100 ug/ml spectinomycin. Colonies growing on this medium were purified and identified as S. flexneri 5 by agglutination with a specific rabbit antiserum. Since pHS6202 cannot multiply in M90T, these clones were expected to have undergone a double recombination event leading to allelic exchange between the DNA sequences flanking ompR and envZ in M90T and the corresponding DNA in pHS6202. One of the resulting mutants, SC433, was selected for further studies. Total chromosomal DNA was prepared from M90T and SC433 and analyzed by hybridization with the three probes (a, b and C) of Example 1. SC433 DNA hybridized with the omega probe (c) and the large ompR-envZ probe (a) containing the flanking sequences but showed no hybridization with the envZ internal probe (b). M90T DNA hybridized with both ompR-envZ probes (a and b) but did not hybridize with the omega probe (c).

By P1 transduction, the ΔompB mutation was transduced from strain SC433 to E. coli strain pop1010 (AroB⁻, Mal+) (17). Since malA, ompB, aroB loci map within 1 min. on the E. coli chromosome, the SpR pop1010 transductants, which had supposedly received the ΔompB mutation, were tested for these phenotypes. The results showed that the SpR marker was cotransducible at a rate of 50 to 60% with each of these loci. Taken together, these data indicated that mutant SC433 was missing the ompB locus and that the location of this locus on the Shigella chromosome was similar to that observed on the E. coli chromosome.

SC433 was tested for expression of virulence in the HeLa cell invasion assay. It showed a significantly reduced invasive phenotype (Table 4), and like the envZ::Tn10 mutant, its rate of intra-cellular multiplication was 1 to 2 logs below that of the wild type strain M90T. It was also negative in the plaque assay and elicited a weak response in the Sereny test. Immunoblots, performed using the serum of a monkey convalescing from shigellosis, showed that the Ipa protein profile of SC433 was not altered.

Further characterization of the ΔompB mutant was carried out after transduction of the vir::lac operon fusion from BS184 into SC433. The resulting strain, SC434, was tested for osmolarity regulated expression of $\beta$-galactosidase. This strain did not show any expression of $\beta$-galactosidase in low or in high osmolarity medium (Table 2).

In addition, the ΔompB mutation was transduced from SC433 to the wild type strain M90T. Two Sp$^R$ transductants, SC435 and SC436, were selected for virulence tests (Table 4). Both transductants were invasive but did not form plaques and gave a mild response in the Sereny test (4). As expected, transcomplementation performed on strains SC433, SC435 and SC436 with plasmid pAT003 restored the wild type phenotype of all these mutants and particularly their capacity to provoke a positive response in the plaque assay and in the Sereny test. This confirmed that the phenotype observed in SC433 resulted from the deletion of the ompR-envZ genes and that these genes are involved in Shigella virulence.

Example 5: Genetic characterization of M90TX -- a spontaneous avirulent ompB mutant tested as a control

If ompB is involved in the regulation of Shigella virulence factors, it may be possible, as observed with Bordetella (34), to obtain spontaneous mutants with reduced virulence which would map at this locus. In 1965, Formal et al (1) isolated a spontaneous avirulent colonial variant of strain M90T. This strain, named M90TX, forms flat and transparent colonies on agar plates. It can be used as a recipient for genetic material from an E. coli K-12 donor strain whereby the Mal$^+$ tranconjugants have their virulence completely restored. Moreover, virulent hybrids strains have incorporated other combinations of genes governing different phenotypes (i.e., Mal, Xyl or Lac, Aro, Mal or lac, Nic, Mal or Aro, Mal). The Pcr$^-$ phenotype of M90TX is generally due to the complete loss of, or a deletion within, the 220 kb virulence plasmid pWR100 (33).

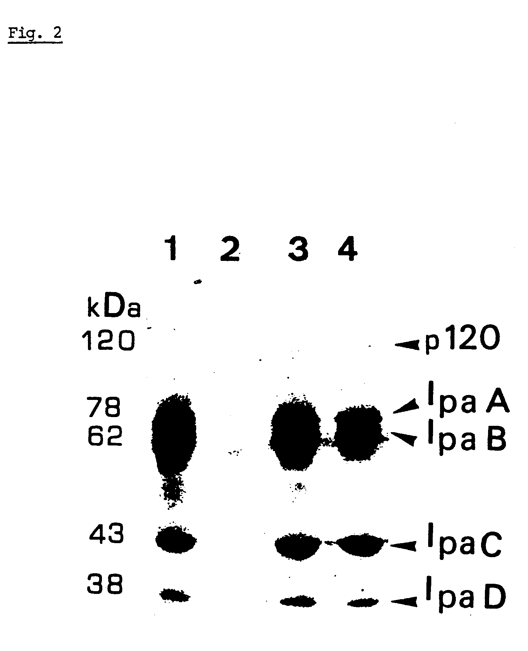

DNA analysis of the 220 kb virulence plasmid of M90TX showed that was unaltered. Virulence tests performed on M90TX showed that it was poorly invasive (5-10% of HeLa cells of the monolayer were infected, as compared to 85% with the wild type strain) and consequently negative in the plaque assay. It also elicited only a very weakly positive or negative result in the Sereny test. Immunoblotting, performed with the serum of a monkey convalescing from shigellosis, showed that this mutant presented an altered expression of Ipa proteins associated with invasion as showed in Fig. 2. Transduction of M90TX, utilizing a P1 lysate raised on E. coli K-12 JM101, restored the virulent phenotype along with the Mal$^+$ character. Therefore, M90TX hybrids and approximately 70% of the transductants, which have incorporated the E. coli chromosomal region around malA (75' min), could be restored to full virulence.

M90TX, therefore, appeared to be a good candidate for a spontaneous mutation in the ompB locus. Indeed pAT003, the ompB recombinant plasmid, restored all the following characteristics: colonial morphology, Pcr$^+$ phenotype and protein profile (Fig. 2), and virulence was similar to that of the wild type phenotype. In contrast, the pW007 recombinant (ompR) plasmid (22) was not able to transcomplement M90TX. This demonstrated that the mutation in M90TX was localized in the ompB locus.

By P1 transduction, the vir::lac operon fusion was transferred into M90TX. Km$^R$ transductants showed a lactose negative phenotype (Lac$^-$) on MacConkey agar plates. One of the transductants, SC438, was tested for osmolarity-regulated expression of $\beta$-galactosidase both in low and high osmolarity media (Table 2) and was found to produce low levels of $\beta$-galactosidase in both media. SC438 carrying the pAT003 plasmid was restored to wild type vir::lac expression in low osmolarity medium. In contrast, induction of vir::lac under conditions of high osmolarity was not observed.

The vir::lac operon fusion was also transferred from BS184 (13) to SC437, a Mal$^+$ virulent transductant of M90TX. This resulted in the Mal$^+$ Km$^R$ strain SC439 which presented similar modulation of $\beta$-galactosidase activity in response to osmolarity changes as BS184, the parental strain (Table 2).

These data suggest that it is not always possible to restore the physiological osmolarity-dependent fluctuations of gene expression with the ompR-envZ products expressed by multicopy recombinant plasmids. In an attempt to test if M90TX (Vir$^-$) could spontaneously revert to the wild type (Vir$^+$) phenotype, the Lac$^-$ vir::lac transductant SC438 was used. Phenotypic Lac$^+$ revertants were isolated at a frequency of approximately $5 \times 10^{-7}$. Two revertants, SC430 and SC431, were analyzed for $\beta$-galactosidase expression and found to have reverted to the wild type phenotype with enhanced vir::lac transcription in high osmolarity medium (Table 2).

Example 6: Determining whether ompF gene expression is modulated by changes in osmolarity

It was demonstrated in previous studies (34) that different missense mutations in E. coli ompB locus could determine different phenotypes with regard to the expression of ompC/ompF in response to fluctuations in osmolarity. Therefore, ompF expression in M90TX was compared to that in other genetically well-characterized E. coli ompB mutants (35). The ompF'-lacZ$^+$ gene fusion from E. coli K-12 JMS150 was transduced into M90T and M90TX, thus producing SC442 and SC443, respectively. SC442 showed the same modulation of ompF expression as strain JMS150. In high osmolarity medium, the ompF'-lacZ$^+$ expression in SC442 decreased by approximately three fold. SC443 showed a markedly increased level of ompF'-lacZ$^+$ expression in low osmolarity medium which was subject to osmolarity-dependent transcriptional regulation (Table 3).

The malA region was transferred by P1 transduction from E. coli K-12 JM101 into SC443, thus generating strain SC444 (Mal$^+$, Lac$^+$). SC444 showed that ompF expression of the lac phenotype was restored to wild type levels in low, as well as in high, osmolarity conditions.

Example 7: Construction and characterization of a ΔompF mutant of M90T

Utilizing the procedures of Examples 1 and 4, a plasmid, like pHS6202, is constructed. The plasmid contains the ompF operon of E. coli K-12, from which a significant portion (about 1 kb) of the porin-encoding DNA sequences of the ompF gene is deleted and is replaced by omega between the flanking sequences of the ompF gene. The plasmid is used for in vivo mutagenis of M90T. The resulting ΔompF mutant shows a significantly reduced invasive phenotype, similar to that of the ΔompB mutant of Example 4.

Conclusions from Examples 1-7

So far, two independent categories of genetic systems, allowing bacteria to respond to environmental conditions, have been studied. In E. coli and closely related enterobacteriaceae, control of DNA supercoiling seems to regulate transcription of a number of genes in response to environmental factors such as osmolarity, anaerobiosis and temperature (36). Recent evidence indicates that virR (13), a gene which is located at 27 min on the chromosome of S. flexneri and which controls cell invasion according to growth temperature, might regulate transcription of the invasion genes via modulation of the supercoiling of the 220 kb virulence plasmid. On the other hand, in E. coli, the ompB locus is comprised of two genes which act coordinately to transduce environmental signals, among which osmolarity is the best studied (21). envZ encodes a transmembrane sensory component interacting with the product of ompR which is a transcriptional regulator (37, 38). Equivalent systems have been characterized which regulate the virulence of human pathogenic bacteria such as Bordetella pertussis (34, 39), Pseudomonas aeruginosa (40) and Vibrio cholerae (41). These are pathogens which colonize mucosal surfaces but do not invade human tissues. It has also been shown that in the invasive pathogen Salmonella typhimurium, phoR-phoB, a two-component system responding to phosphate concentration, controls virulence, via resistance of the bacterium to killing by macrophages (42, 43).

Although, all these systems share homology (44), the environmental factors to which they respond, especially in vivo, are not fully characterized. The foregoing Examples have taken advantage of the homology existing between E. coli and S. flexneri to demonstrate that the Shigella equivalent of the ompB locus of E. coli plays a role in the expression of the invasive phenotype which characterizes this species. Complete deletion of the ompB locus via allelic exchange with an in vivo mutagenized equivalent locus of E. coli provided a mutant which expressed a low level of entry into cells and a limited capacity to survive intracellularly. More definitive virulence assays have confirmed that the virulence of this mutant was severely impaired since the Sereny test (4), which provided a delayed, transient and attenuated response, could be considered as negative. Transduction experiments allowed the S. flexneri mutation to be mapped close to the malA and aroB loci. DNA hybridization analysis, performed in stringent conditions, indicated that the actual ompB locus of S. flexneri had been deleted.

The data in the foregoing Examples confirmed that the complementation experiments carried out with the ompB locus of E. coli did not reflect an indirect "cross-talk" effect. Rather, ompB appears to regulate both plasmid genes which encode the entry process, as well as chromosomal genes necessary for intracellular multiplication, among which porin-encoding genes seem to be the major targets. The latter are "house keeping" genes which become virulence genes after the bacterium has invaded a cell and needs to obtain nutrients from the intra-cellular compartment.

Transduction of an envZ::Tn10 mutation into S. flexneri resulted in a virulence phenotype similar to that of the ompB deletion mutant. However, since the envZ gene is transcribed downstream of ompR, it has been impossible to identify the actual function of ompR. Due to a polar effect, ompR mutants are also envZ mutants. In addition, it has not been possible to restore conditions in which the envZ product could be produced at a physiological level (10 molecules per cell) in an ompR deletion mutant.

A vir::lac fusion, introduced into the wild type strain M90T, permitted the study of vir expression by monitoring the level of β-galactosidase activity as opposed to invasive ability. In this fusion mutant, passage from low to high osmolarity was correlated with a 3 to 4 fold increase of β-galactosidase production. This is similar to the range of variation observed with ompC and ompF in equivalent conditions (45). Although osmotic pressures (300 mOsm) which induce submaximal expression of the vir::lac fusion are found in the intra- and extracellular compartments of human hosts, introduction of a vir::lac fusion into strains harboring either the envZ::Tn10 or the ΔompB mutations lowered the expression of β-galactosidase. Induction of the enzymatic activity was not, however, eliminated under conditions of high osmolarity. This suggests that, although envZ is necessary for optimal expression of the virulence genes, it is not sufficient, by itself, to respond to a switch to high osmolarity.

Full virulence was restored to naturally occurring ompB mutants of S. flexneri, which had been isolated as an avirulent colonial variant (1), by transcomplementation with the envZ-ompR genes of E. coli but not with ompR alone. In addition, the M90TX phenotype was not the same as that of the ΔompB Shigella mutant. It was pleiotropic with respect to several different phenotypes as had been previously observed in well characterized envZ mutations of E. coli (17, 47, 48) which suggests that a spontaneous mutation might have occurred in the envZ sequence. M90TX was even less invasive than the envZ::Tn10 and ompB mutants. In this regard, production of Ipa proteins was dramatically reduced, and the strain did not bind the Congo red dye. M90TX showed an increased expression of ompF in low osmolarity medium as monitored by a ompF::lacZ gene fusion, although it was still able to reduce ompF expression when grown in high osmolarity medium. When the vir::lac fusion was introduced into M90TX, no expression of β-galactosidase was observed.

Phenotypic reversion of osmotically regulated expression of β-galactosidase was obtained at a frequency of $5 \times 10^{-7}$. This could indicate that, as in B. pertussis, a mechanism of phase variation exists in Shigella at the level of virulence genes. The relevance of phase variation in S. flexneri ecology and pathogenicity is unknown.

The data in the Examples also suggest that environmental regulation of Shigella, particularly S. flexneri, invasiveness involves a very subtle mechanism which involves at least two types of systems:

1) A stringent temperature regulation system which swiches virulence on and off; this may reflect the necessity for bacteria to become rapidly invasive when sensing mammalian body temperature that indicates that they have reached their target-host; and

2) a less stringent system of adaptation to different extra- or intra-cellular compartments within their target-host; this system may necessitate a more subtle modulation, for which control by the envZ and ompR genes is better adapted.

It is believed that this invention and many of its attendant advantages will be understood from its description, above, and it will be apparent that various modifications can be made in the mutant Shigellae the method of obtaining them, and the vaccine containing them, as described above, without departing from the spirit and scope of the invention or sacrificing all of its material advantages, the embodiments described above being merely preferred embodiments.

The references, referred to above, are as follows.

**REFERENCES.**

1. Formal, B. S., La Brec, E. H., Schneider, H. & Falkow, S. (1965) *J. Bacteriol.* **89**, 835-838.
2. La Brec, E. H., Schneider, H., Magnani, T. J. & Formal, S. B. (1964) *J. Bacteriol.* **88**, 1503-1518.
3. Oaks, E. V., Hale, T. L. & Formal, S. B. (1986) *Infect. Immun.* **48**, 124-129.
4. Sereny, B. (1955) *Acta Microbiol. Acad. Sci. Hung.* **2**, 293-296.
5. Sansonetti, P. J., Hale, T. L., Dammin, G. J., Kapfer, C., Collins, H. H. Jr. & Formal, S. B. (1983) *Infect. Immun.* **39**, 1392-1402.
6. Maurelli, A. T., Baudry, B., d'Hauteville, H., Hale, T. L. & Sansonetti, P. J. (1985) *Infect. Immun.* **49**, 164-171.
7. Bernardini, M. L., Mounier, J., d'Hauteville, H., Coquis-Rondon, M. & Sansonetti, P. J. (1989) *Proc. Natl. Acad. Sci. USA* **86**, 3867-3871.
8. Makino, S., Sasakawa, C., Kamata, K., Kurata, T. & Yoshikawa, M. (1986) *Cell* **46**, 551-555.

9. Sansonetti, P. J., Kopecko, D. J. & Formal, S. B. (1981) *Infect. Immun.* **35**, 852-860.

10. Miller, J. M., Mekalanos, J. J. & Falkow, S. (1989) *Science* **243**, 916-922.

11. Ronson, C. W., Nixon, B. T. & Ausubel. F. M. (1987) *Cell* **49**, 579-581.

12. Comeau, D. E., Ikenaka, K., Tsung, K. & Inouye, M. (1985) *J. Bacteriol.* **164**, 578-584.

13. Maurelli, A. T. & Sansonetti, P. J. (1988) *Proc. Natl. Acad. Sci. USA* **85**, 2820-2824.

14. Casadaban, M. J. (1976) *J. Mol. Biol.* **104**, 541-555.

15. Slauch, J. M., Garrett. S., Jackson, D. E. & Silhavy, T. J. (1988) *J. Bacteriol.* **170**, 439-441.

16. Yanish-Perron, C., Vieira, J. & Messing, J. (1985) *Gene* **33**, 103-119.

17. Wandersman, C., Moreno, F. & Schwartz, M. (1980) *J. Bacteriol.* **143**, 1374-1383.

18. Miller, V. J. & Mekalanos, J. J. (1988) *J. Bacteriol.* **170**, 2575-2583.

19. Casadaban, M. (1980) *J. Mol. Biol.* **138**, 179-183.

20. Sansonetti, P. J., Kopecko, D. J. & Formal, S. B. (1982) *Infect. Immun.* **35**, 852-860.

21. Mizuno, T., Wurtzel, T. E. & Inouye, M. (1982) *J. Bacteriol.* **150**, 1462-1466.

22. Wurtzel, T. E., Movva, R. N., Ross, F. & Inouye, M. (1981) *J. Mol. Appl. Genet.* **1**, 61-69.

23. Miller, J. (1972) Experiments in Molecular Genetics. (Cold Spring harbor Lab., Cold Spring Harbor, N. Y.)

24. Dagert, M. & Ehrlich, S. D. (1979) *Gene* **6**, 23-28.

25. Kawaji, H., Mitzuno, T. & Mirushima, S. (1979) *J. Bacteriol.* **140**, 843-847.

26. Hale, T. L. & Formal, S. B. (1981) *Infect. Immun.* **32**, 137-144.

27. Burnette, W. N. (1981) *Anal. Biochem.* **112**, 195-203.

28. Ish-Horowitz, D. & Burke, J. F. (1981) *Nucl. Acids. Res.* **9**, 2989-2994.

29. Silhavy, T. J., Berman, M. L. & Enquist, W. L. (1984) Experiments with gene fusions (Cold Spring Harbor Lab, Cold Spring harbor, N. Y.)

30. Prentki, P. & Kirsck, M. M. (1984) *Gene* **29**, 303-313.

31. Fontaine, A., Arondel, J. & Sansonetti, P. J. (1988) *Infect. Immun.* **56**, 3099-3109.

32. Southern, E. M. (1975) *J. Mol. Biol.* **98**, 503-517.

33. Maurelli, A. T., Blackmon, B. & Curtis III, R. (1984) *Infect. Immun.* **43**, 397-401.

34. Stibitz, S., Aaronson, W., Monack, D. & Falkow, S. (1989) *Nature* **338**, 266-269.

35. Forst, S. & Inouye, M. (1988) *Ann. Rev. Cell. Biol.* **4**, 21-42.

36. Bhrian, N. N., Dorman, C. J. & Higgins, C. F. (1989) *Mol. Microbiol.* **3(7)**, 933-942.

37. Norioka, S., Ramakishnan, G., Ikenaka, K. & Inouye, M. (1986) *J. Biol. Chem.* **261**, 17113-17119.

38. Forst, S., Comeau, D., Shigemi, N. & Inouye, M. (1987) *J. Biol. Chem.* **262**, 16433-16438.

39. Arico, B., Miller, F. J., Craig, R., Stibitz, S., Monack, D., Falkow, S., Gross, R. & Rappuoli, R. (1989) *Proc. Natl. Acad. Sci. USA* **86**, 6671-6675.

40. Dertic, V., Dikshit, R., Konyescni, W. M., Chakrabarty, A. M. & Misra, T. K. (1989) *J. Bacteriol.* **171**, 1278-1283.

41. Miller, V. L., Taylor, R. K. & Mekalanos, J. J. (1987) *Cell* **48**, 271

42. Groisman, E. A., Chiao, E., Lipps, C. J. & Heffron, F. (1989) *Proc. Natl. Acad. Sci. USA* **86**, 7077-7081.

43. Miller, S. I., Kukral, A. M. & Mekalanos, J. J. (1989) *Proc. Natl. Acad. Sci. USA* **86**, 5054-5058.

44. Gross, R., Arico, B. & Rappuoli, R. (1989*) Mol. Microbiol.* **3(11)**, 1661-1667.

45. Jovanovich, S. B., Martinell, M., Record, Jr. M. T. & Burgess, R. R. (1988) *J. Bacteriol* **170**, 534-539.

46. Adler, B., Sasakawa, C., Tobe, T., Makino, S., Kometsu, K. & Yoshikawa, M. (1989) *Mol. Microbiol.* **3-(5)**, 627-636.

47. Taylor, R. K., Hall, M. N. & Silhavy, T. J. (1983) *J. Mol. Biol.* **166**, 273-282.

48. Matsuyama, S., Mizumo, T. & Mirushima, S.(1986) *J. Bacteriol.* **168**, 1309-1314.

**TABLE 1: BACTERIA AND PLASMIDS.**

| STRAINS | GENOTYPE AND RELEVANT CHARACTERISTICS | REFERENCE OR SOURCE |
|---|---|---|
| *E. coli* K-12 | | |
| MC4100 | $F^-$ *araD139* $\Delta$*(argF-Lac)U169 rps L150 relA1 flbB-5301 ptsF25 deoC1 thiA1* | (14) |
| JMS60 | MC4100 *envZ60::Tn10*, Tc$^R$ | (15) |
| JMS150 | MC4100 (*ompF'-lac*R) 16-13 | (Slauch-Silhavy) |
| JM101 | *supE, thi, $\Delta$ (lac-proAB) F', traD36, proAB, lacI$^q$ Z$\Delta$M15* | (16) |
| pop1010 | Hfr, *his, metA, aroB, rpoB* | (17) |
| SM10$\lambda pir$ | *thi, thr, leu, tonA, lacY, supE, recA::RP4-2-Tc::Mu*, Km$^R$; contains the prophage $\lambda pir$ which encodes the $\Pi$ protein. | (18) |
| MC1061 | *araD139* $\Delta$ *(ara, leu)* 7697, $\Delta$*lacX74, galU, galR hsr, hsm, strA.* | (19) |

*S. flexneri*

| | | |
|---|---|---|
| BS184 | *S. flexneri* 2a; 2457T (vir-83::MudI 1734), (13) KmR. | |
| M90T | *S. flexneri*5; wild type harboring 220 kb- (20) invasion plasmid pWR100. | |
| M90TX | Spontaneous avirulent variant of M90T. (1) | |
| SC430 | M90T *vir::lac* transductant of BS184 donor; KmR. | This invention |
| SC431 | SC430 *envZ*60::Tn*10* transductant of JMS60 donor; TcR, KmR | " |
| SC432 | M90T *envZ*60::Tn*10* transductant of JMS60 donor; TcR | " |
| SC433 | M90T Δ *ompB*; SpR | " |
| SC434 | SC433 *vir::lac* transductant of BS184 donor; KmR, SpR | " |
| SC435 and SC436 | M90T Δ *ompB* transductants of SC433 donor; SpR | " |
| SC438 | M90T *vir::lac* transductant of BS184 donor, KmR | " |
| SC437 | M90TX *malA*, *ompB* transductant of JM10 donor | " |
| SC439 | SC437 *vir::lac* tranductant of BS184 donor; KmR | " |
| SC440 and SC441 | Spontaneous phenotypic Lac+ revertants of SC438; KmR | " |

SC442          M90T *ompF'-lacZ* transductant of JMS150

                       donor                                    "

SC443          M90TX *ompF'-lacZ* transductant of JMS150

                       donor                                    "

SC444          SC443 *malA, ompB* transductant of JM101

                       donor                                    "

PLASMIDS

pJM7031        OriR6K, Rep$^-$, Mob$^+$, Ap$^R$            (18)

pAT003         vector pBR322                     (21)

                cloned *E. coli* genes *ompR-envZ*, Ap$^R$

pW007          vector pBR322                     (22)

                cloned *E. coli* gene *ompR*, Ap$^R$

pHS6200        pAT003 missing the EcoRI,

                restriction site of pBR322 sequence, Ap$^R$;  "

pHS6201        pHS6201 cloned Δ *ompB*-Ω, Ap$^R$ Sp$^R$      This invention

pHS6202        vector pJM703.1,

                cloned Δ *ompB*-Ω, Ap$^R$ Sp$^R$                 "

TABLE 2

| EXPRESSION OF $\beta$-GALACTOSIDASE ACTIVITY FROM THE *vir*-83::Mu dl1734 OPERON FUSION. | | | |
|---|---|---|---|
| STRAIN | $\beta$-galactosidase units. | | RATIO MA + 15%/MA |
| | MEDIUM A | MEDIUM A + 15% SUCROSE | |
| BS184 | 200 | 700 | 3.5 |
| SC430 | 183 | 667 | 3.6 |
| SC431 | 80 | 336 | 4 |
| SC434 | 20 | 18 | 0,9 |
| SC438 | 50 | 46 | 0,9 |
| SC438 pAT003 | 200 | 180 | 0,9 |
| SC439 | 173 | 674 | 3.9 |
| SC440 | 190 | 720 | 3.7 |

14

TABLE 3

| EXPRESSION OF $\beta$-GALACTOSIDASE ACTIVITY FROM THE (*ompF'-lacZ*+) 16-13 GENE FUSION. | | | |
|---|---|---|---|
| STRAIN | $\beta$-Galactosidase units. | | RATIO MA/MA + 15% |
| | MEDIUM A | MEDIUM A + 15% SUCROSE | |
| JMS150 | 277 | 86 | 3.2 |
| SC442 | 328 | 91 | 3.6 |
| SC443 | 630 | 206 | 3.0 |
| SC444 | 257 | 83 | 3.1 |

TABLE 4

| EXPRESSION OF THE VIRULENCE-ASSOCIATED PHENOTYPES IN WILD TYPE AND MUTANT STRAINS OF S. *FLEXNERI* 5. | | | | |
|---|---|---|---|---|
| STRAINS | HELA CELL INVASION* | PLAQUE ASSAY | SERENY** TEST | CONGO RED BINDING |
| M90T | 85% | + | + | + |
| SC432 | 40% | - | - | + |
| SC433 | 30% | - | - | + |
| M90TX | 5% | - | - | - |
| M90TX pAT003 | 70% | + | + | + |
| SC437 | 90% | + | + | + |
| SC435 and SC436 | 30% | - | - | + |

* Percentage of infected HeLa cells.
** A Sereny test was considered as positive when a strong keratoconjunctivitis occured within 48h after inoculation.

**Claims**

1. A method for making a vaccine against a wild strain of an entero-invasive Shigella, such as S. flexneri, by providing a mutant of Shigella strain having a modified genome; the method being characterized by the step of:
   mutagenizing the ompB locus of the strain so that at least a portion of its ompR gene and at least a portion of its envZ gene are removed or permanently inactivated.

2. The method of claim 1 characterized by the additional step of mutagenizing a second gene of the strain, encoding a protein necessary for the strain to: invade and then multiply within infected cells of a host; spread within infected cells and from infected to uninfected cells of the host; and/or produce toxins which will kill the host's infected and uninfected cells.

3. The method of claim 2 in which the second gene is an icsA gene.

4. The method of claim 3 characterized by the further step of mutagenizing a third gene encoding a protein necessary for the chelation and/or transport of iron in the strain.

5. The method of claim 4, wherein the strain is an S. dysenteriae 1, characterized by the still further step of mutagenizing a fourth gene coding for the production of Shiga-toxin by the S. dysenteriae 1.

6. The method of any one of claims 1-5, wherein one or more of the genes are mutagenized by allelic exchange with one or more in vitro mutagenized genes, especially mutagenized genes from which portions have been deleted and particularly mutagenized genes into which marker genes have been inserted.

7. A Shigella which has been modified by the method of any one of claims 1-6 or is a descendant thereof.

8. A vaccine against Shigella, made by the method of any one of claims 1-6.

**Patentansprüche**

1. Verfahren zur Herstellung eines Impfstoffs gegen einen entero-invasiven Wildtypstamm von Shigella, beispielsweise S. flexneri, durch Bereitstellung einer Mutante eines Shigellastamms mit einem modifizierten Genom, wobei das verfahren durch folgenden Schritt gekennzeichnet ist:
   Mutagenisieren des ompB-Locus des Stamms, daß mindestens ein Teil seines ompR-Gens und mindestens ein Teil seines envZ-Gens entfernt oder permanent inaktiviert werden.

2. Verfahren nach Anspruch 1, dadurch gekennzeichnet, daß in einem zusätzlichen Schritt ein zweites Gen des Stammes mutagenisiert wird, das ein Protein codiert, welches für den Stamm notwendig ist, damit dieser eindringen und im Anschluß daran sich innerhalb der infizierten Zellen eines Wirts vermehren kann; sich innerhalb der infizierten Zellen und sich von infizierten auf nicht infizierte Zellen des Wirts ausbreiten kann; und/oder Toxine herstellen kann, die die infizierten und nicht-infizierten Zellen des Wirts töten.

3. Verfahren nach Anspruch 2, wobei das zweite Gen ein icsA-Gen ist.

4. Verfahren nach Anspruch 3, dadurch gekennzeichnet, daß in einem weiteren Schritt ein drittes, ein für die Chelatbildung und/oder den Transport von Eisen in dem Stamm notwendiges Protein codierendes Gen mutagenisiert wird.

5. Verfahren nach Anspruch 4, wobei der Stamm S. dysenteriae 1 ist, dadurch gekennzeichnet, daß in noch einem weiteren Schritt ein viertes für die Herstellung von Shiga-Toxin durch S. dysenteriae 1 codierendes Gen mutagenisiert wird.

6. Verfahren nach einem der Ansprüche 1 bis 5, wobei ein Gen oder mehrere Gene durch Allelaustausch mit einem oder mehreren in vitro mutagenisierten Gen(en) mutagenisiert werden, insbesondere mutagenisierte Gene, von denen Bereiche deletiert wurden und bevorzugt mutagenisierte Gene, in die Markergene inseriert wurden.

7. Shigella, modifiziert durch das Verfahren nach einem der Ansprüche 1 bis 6 modifiziert, oder ein Nachkomme davon.

8. Impfstoff gegen Shigella, hergestellt durch das Verfahren nach einem der Ansprüche 1 bis 6.

**Revendications**

1. Procédé de fabrication d'un vaccin contre une souche sauvage d'une Shigella entéro-envahissante, telle que S. flexneri, en fournissant un mutant de souche de Shigella ayant un génome modifié; le procédé étant caractérisé par l'étape consistant à :
   soumettre à la mutagenèse le locus ompR de la souche de telle sorte qu'au moins une partie de son gène ompB et au moins une partie de son gène envZ soient enlevées ou inactivées de façon permanente.

2. Procédé selon la revendication 1, caractérisé par l'étape supplémentaire consistant à soumettre à la mutagenèse un deuxième gène de la souche, codant pour une protéine nécessaire pour que la souche: envahisse, puis se multiplie à l'intérieur de cellules infectées d'un hôte; se propage à l'intérieur de cellules infectées et à partir de cellules infectées à des cellules non infectées de l'hôte; et/ou produise des toxines qui tueront les cellules infectées et non infectées de l'hôte.

**3.** Procédé selon la revendication 2 dans lequel le deuxième gène est un gène icsA.

**4.** Procédé selon la revendication 3, caractérisé par l'étape supplémentaire consistant à soumettre à la mutagenèse un troisième gène codant pour une protéine nécessaire à la chélation et/ou au transport du fer dans la souche.

**5.** Procédé selon la revendication 4, dans lequel la souche est une S. dysenteriae 1, caractérisé par encore l'étape supplémentaire consistant à soumettre à la mutagenèse un quatrième gène codant pour la production de toxine de Shiga par S. dysenteriae 1.

**6.** Procédé selon l'une quelconque des revendications 1 à 5, dans lequel un ou plusieurs des gènes sont soumis à la mutagenèse par échange allélique avec un ou plusieurs gènes soumis à la mutagenèse in vitro, notamment des gènes ayant été soumis à la mutagenèse à partir desquels des parties ont été délétées et particulièrement des gènes ayant été soumis à la mutagenèse dans lesquels des gènes marqueurs ont été insérés.

**7.** Shigella ayant été modifiée par le procédé de l'une quelconque des revendications 1 à 6 ou un descendant de celle-ci.

**8.** Vaccin contre Shigella, fabriqué par le procédé de l'une quelconque des revendications 1 à 6.

Fig. 1

Fig. 2